# EUROPEAN PATENT APPLICATION

(11) **EP 1 987 726 A1**
(43) Date of publication of application: **05.11.2008**
(21) Application number: 07107282.1
(22) Date of filing: 01.05.2007
(51) Int. Cl.: A23L 1/30, A23L 1/307, C12N 9/10, A61K 38/45

(54) **Good tasting food product containing a neutralisation agent for adverse compounds**

(71) Applicant: Friesland Brands B.V., 7943 PE Meppel (NL)
(72) Inventor: Bongers, Cornelis Margaretha Theodorus Maria, 5707 KR Helmond (NL); Dekkers, Renske, 6824 DS Arnhem (NL); Looijensteijn, Petronella Johanna, 8017 BA Zwolle (NL)
(74) Representative: van Loon, C.J.J.

(57) **Abstract**

The present invention relates to good tasting food products that contain an agent or agents which avoid that taste-essential ingredients having an adverse effect, when digested or when taken up in the system of the consumer, can perform their adverse effects. The said adverse effect is especially an adverse effect on human health. In addition, the present invention relates to particular compounds or compositions that can be used for the directed removal of such adverse components of foods (including drinks) especially in the upper intestinal tract.

## Description

The present invention relates to good tasting food products that contain an agent or agents which avoid that taste-essential ingredients having an adverse effect, when digested or when taken up in the system of the consumer, can perform their adverse effects. The said adverse effect is especially an adverse effect on human health. In addition, the present invention relates to particular compounds or compositions that can be used for the directed removal of such adverse components of foods (including drinks) especially in the upper intestinal tract.

A well-known example of a taste-essential ingredient that also has adverse effects when taken up in the system of a consumer is sucrose. Sucrose is thé standard for a sweet taste, but has a high caloric value and, associated therewith, may lead to undesirable weight gains, obesity, diabetes type II *etc..*

Another example of such an ingredient is lactose which also imports a pleasant sweetness, yet many persons suffer from lactose intolerance.

Yet a further example are fats. Fats *per se* add to a pleasant mouth feel, and carry many important taste and aroma compounds that are fat or oil soluble. Fats are, however, also calorie rich.

The Chinese restaurant syndrome is caused by mono sodium glutamate or ve-tsin, which compound is a taste enhancer, but at the same time is not tolerated by a high number of people.

As a last, yet non-limiting example, reference is made to proteins which may give rise to allergic reactions, but have a negative off-flavour when added in the form of hypo allergic peptides. In this light, it is noted that e.g. hydrolyzed peptides often have a bitter taste.

Quite a number of the adverse effects are associated with medical indications, such as obesitas, diabetes, allergic reactions and intolerances.

In the state of the art, a high number of products are described wherein the components having adverse effects are replaced by other harmless components. Sucrose may for example be replaced by artificial sweeteners. Oftentimes, however, replacement of the taste essential ingredient leads to a less pleasant taste of the food product.

Also when part of the taste essential component is replaced by a taste intensifier, oftentimes less tasteful products result or the taste profile of a product changes.

The present invention has the object to provide a food, inclusive a drink, that maintains its pleasant taste, in the sense that the taste essential component maintains its sensoric availability, while the adverse effects thereof are reduced, alleviated, neutralized or sometimes even converted in favorable effects. That is, after providing their favorable taste effect, the components involved are converted in harmless and sometimes even health positive components.

### State of the Art.

EP-0 457 919 relates to functional foods or edible compositions having a calorie intake lowering function. These compositions comprise at least one polysaccharide producing enzyme from the group consisting of glucosyl transferases and fructosyl-transferases having water soluble polysaccharide production ability. The enzymes form polysaccharides from sucrose in the digestive tract to lower calorie intake and by this mechanism in the digestive tracts of the food compositions described are said to be useful for the prophylaxis of adult disease factors such as obesity. To avoid reduction in activity by a digestive fluid such as the gastric juices when the enzyme is orally ingested, the compositions described are constituted in enteric dosage forms which protect the compositions against deactivation before the digestive tract is reached.

In EP-0 968 719, an enzyme composition is described which is capable of forming an oligosaccharide *in vivo,* which oligosaccharide must exert physiological activity in the living body. In view of the physiological activities aimed at, the document indicates that "it is desirable that these oligosaccharides are formed at the initial stage of the digestive tracts" and in this light, it is described that the enzyme composition is capable of exerting its action in the stomach.

WO-A-2004/080200 is directed to probiotic food compositions. These compositions contain spores of bacteria or fungi or contain enzymes, which active components should enhance the digestion of carbohydrates and/or proteins. To increase the probability of survival in gastro-intestinal conditions (low pH; bile) the vegetative forms of microorganisms may be encapsulated or micro-encapsulated.

### Invention

The present invention solves the above-identified object by combining the taste essential component with a microorganism and/or an enzyme, which microorganism and/or enzyme is capable of converting the taste essential component in a component or components which do not have the adverse effect or only have said effect in a lesser degree, said taste essential component and said microorganism and/or enzyme being present in said food without subtantially reacting with each other before being consumed.

In a first embodiment, the invention hence relates to a food comprising (i) at least one taste essential ingredient that has an adverse effect when taken up in the system of a consumer and (ii) at least one enzyme capable of converting ingredient (i) in a component or components not having said adverse effect or at least having said adverse effect in a decreased degree; wherein ingredient (i) and enzyme (ii) do not react with each other before consumption.

In a preferred embodiment, the enzyme is produced by a microorganism present in said food composition. Preferentially, said microorganism is a living microorganism, and more preferably a probiotic microorganism. Suitably, such a microorganism is selected from the genera *Lactobacillus, Bifidobacterium,* and *Leuconostoc.*

In yet a further preference, the enzyme is an exocellular enzyme.

As an alternative, the enzyme is obtained from or in the form of a lysate of a microorganism.

In case in the remainder of this description, reference is made to "microorganism" as being a component of the food composition of the present invention, then the intention is to refer to said microorganism as source of the enzyme that is an essential ingredient of the said food composition according to the invention.

The reaction between ingredient (i) and microorganism and/or enzyme (ii) only starts when the food, inclusive any drinks, is consumed. That is, the reaction takes place in the time span between the moment that the food or drink is in the mouth and the moment right before the taste essential component can create it adverse effect, for example the moment wherein the said ingredient is absorbed in the intestines. It speaks for itself that dependent on the taste essential ingredient, the time period before said ingredient needs to be adapted (or the length of the route through the body before said ingredient reaches the site where it may lead to its adverse effect) may vary.

By way of example, sucrose manifests its sweetness creating effect in the mouth, yet is converted in harmless or less harmful components before it is taken up in the small intestines after which it may manifest its adverse effects as high calorie molecule and/or in diabetes type II.

With "taste essential ingredient" is meant that an ingredient acceptable for use in a food, including a drink, has some characteristic effect on the taste of a product containing said ingredient. This characteristic may be a taste, an aroma, an effect on the mouthfeel or any other perceivable sensoric effect. That is, a taste essential ingredient adds to the sensoric perception of a food.

In a preferred embodiment, said taste essential ingredient is selected from the group consisting of a carbohydrate and especially a sugar, a fat, a protein, glutamate, and any mixture thereof.

As a non-limiting example of a food ingredient that is essential for the taste of the food product, yet has adverse effects when taken up in the system of the consumer, sugars, and especially sucrose, will be discussed.

Carbohydrates are an important, if not the most important, source of energy in a food product and are found in many kinds in every day's food compositions. Within the carbohydrate family, sugars, and especially mono-and disaccharides, provide a pleasant sweet taste. To reduce the energetic or caloric value of sugars-, and especially of sucrose-containing compositions often artificial sweeteners are used. Such sweeteners however have oftentimes an adverse effect on the taste of the food product incorporating these. Moreover, large amounts of polyols (like xylitol) may give rise to flatulence or other systemic inconveniences. Furthermore, not all sweeteners are accepted by consumers nor by governmental authorities in all countries. In addition, the perception in taste may differ from consumer to consumer, while many consumers indicate that artificial sweetener containing compositions suffer from an unpleasant, for instance bitter or metallic, after-taste.

Food compositions having a high carbohydrate content may be categorized on the basis of the so-called glycemic index. This numeric index is based on the average increase of the glucose level in the blood of the consumer after eating a particular amount of the food composition. If a food composition contains a high amount of carbohydrates, then this does not necessarily mean that said composition automatically has a high glycemic index, since eating said composition may not lead to a higher glucose level. Instead of referring to the glycemic index, one also refers to the effective energetic value of a particular food composition. This value relates to the part of the energetic value of the food composition that is effectively absorbed by the system and leads to an increased glucose level in the blood.

The effective energetic value of a sugar containing food composition may be decreased when steps are taken to avoid or inhibit that the sugars are absorbed in the gastro-intestinal tract.

One way of achieving this is by inhibiting the hydrolytic activity of the enzyme sucrase in the intestines. Sucrase is an enzyme converting the disaccharide sucrose, also known as saccharose, in the monosaccharides fructose and glucose. An example of a sucrase inhibitor is L-arabinose.

Another known possibility is to convert ingestible sugars in non-digestable molecules like oligosaccharides or sugar alcohols. Sugar alcohols are partially absorbed in the ileum, yet do not take part in the systemic metabolism and are excreted via the kidneys. The remaining part is slowly fermented in the colon, where these compounds may have a prebiotic activity, meaning that the growth and activity of certain beneficial bacteria in the intestinal flora, such as *Lactobacilli* and *Bifidobacteria,* are stimulated. Oligosaccharides have a corresponding behaviour in the intestines.

Particularly, commonly used digestible sugar molecules which may be converted by the microorganism and/or enzyme comprise, for example, glucose, sucrose, fructose, lactose, and galactose; the said sugar molucules are converted to indigestible molecules like glucose polymer, fructose polymer, fructo-oligosaccharides (FOS), mannitol, and galacto-oligosaccharides (GOS). When, for instance, FOS or GOS is formed, it is known that such compounds have a prebiotic activity; that is, these compounds stimulate the growth and activity of benigne bacteria, and especially of *Lactobacilli* and *Bifidobacteria,* in the intestinal flora. Hence, in a preferred embodiment of the present invention, at least part of the sugar molecules present in the food products of the invention are converted in oligosaccharides having prebiotic activity.

The above-cited EP-A-0 968 719 teaches an enzyme composition comprising amylase and enzymes capable of forming sugar alcohols and/or oligosaccharides, and additionally teaches to add such a composition to a food composition. However, when the enzyme composition is contacted with the food composition before consumption, the enzyme catalyzed reaction will start; dependent on the food composition this reaction may be more or less fast, but will have a negative effect on the taste of the food composition.

The main differences between this known embodiment and the present invention are at least two-fold. In accordance with the present invention, the reaction between the taste essential ingredient and the specific microorganism and/or enzyme is delayed until after the taste essential ingredient has achieved its taste effect; that is, the taste essential ingredient will be sensorically available, and only afterwards be converted or neutralized. Further, the amounts of the taste essential ingredient and the microorganisms and/or enzymes are mutually adjusted so that the taste essential ingredient is converted or neutralized to a predetermined, or at least acceptable or desired level under systemic conditions.

As an indication, the amount of enzymes or probiotics to be used in a food composition of the present invention generally is at least about 0.2 wt.%, drawn on the mass of their substrate, for instance sugars; more preferably said amount is at least 0.5 wt.% and even more preferably at least about 1 wt.% drawn on the mass of the substrate.

The microorganisms that may be used in the composition of the present invention can be living microorganisms, and are preferably microorganisms excreting exo-enzymes or containing cell-wall anchored enzymes, which enzymes convert the taste essential ingredient. Non-limitative examples are *Leuconostoc* and *Lactobacillus sp.* containing glucan and fructan sucrases that convert sucrose; *Lactobacillus lactis* having a protease that converts proteins; and yeasts producing extracellular lipases that convert fat. In a more preferred embodiment, the microorganisms do not only provide desirable enzymes, but are themselves probiotics.

More in general, it is noted that examples of suitable enzymes to be used in the food compositions of the present invention are glucosyl transferases (for example dextran sucrase), fructosyl transferases (e.g., inulosucrase, levansucrase, fructan:fructan 6G-fructosyltransferase), galactosyl transferase, and mannitoldehydrogenase.

Fructosyltransferases may be produced by the following probiotics: *Lactobacillus reuteri, Lactobacillus acidophilus,* and *Streptococcus salivarius.* Also non-probiotic bacteria such as *Leuconostoc citreum, Leuconostoc mesenteroides* and *Lactobacillus sanfranciscensis* are able to produce fructosyltransferases. A highly preferred enzyme is the levansucrase, for instance prepared by *Lactobacillus reuteri* because it shows a relatively high degree of sucrose conversion. In addition, it is known that fructosyltransferases occur in high concentrations in among others, onions and chicory. The synthesis of fructosyltransferases by bacteria may be increased by keeping the enzymes in a medium containing sucrose.

Mannitoldehydrogenases show a very efficient conversion of fructose to mannitol. These enzymes may be produced by the following probiotics: *Lactobacillus intermedius, Lactobacillus fermentum, Lactobacillus reuteri,* and *Lactobacillus plantarum.* Also non-probiotic bacteria like *Leuconostoc pseudomesenteroides, Leuconostoc mesenteroides* and *Lactobacillus sanfranciscensis* are capable of producing mannitoldehydrogenases. Very suitable mannitol producing lactic acid bacteria are heterofermentative, *i.e.* these bacteria produce in addition to lactate also CO₂ and ethanol. The activity of mannitoldehydrogenases may be increased by keeping the enzymes in a medium containing fructose.

Galactosidases may be produced by the probiotic *Bifidobacterium adolescentis* (α-galactosidase). Also non-probiotic bacteria like *Kluyveromyces fragilis, Aspergillus oryzae* and *Escherichia coli* can produce galactosidases (β-galactosidase). In this light, it is noted that β-galactosidases are preferred because of their prebiotic activity.

Instead of living microorganisms also dead organisms may be used as a kind of reservoir for suitable enzymes. In order to make these enzymes available, it may sometimes be needed to use disrupted or lysated microorganisms.

In the most preferred embodiment, the microorganisms are lactic acid bacteria, such as those of the genera *Leuconostoc* or *Lactobacillus,* especially because these microorganisms are often used in food applications.

Instead of or in combination with microorganisms also enzymes may be used. Such enzymes may be of botanic origin (e.g. of chicory), of microbial origin (e.g. levansucrase from *Zymomonczs mobilis* or transglucosidase from *Aspergillus niger)* or of animal origin.

In one embodiment, the microorganisms and/or enzymes are physically separated from the taste essential ingredient in the food composition, or the two reactants are because of physical and/or chemical conditions not capable to react with each other in the unconsumed food composition.

This may be achieved by incorporating or encapsulating the microorganisms and/or enzymes in a solid, inclusive of gel-type matrix such that interaction with the taste essential ingredient becomes only possible after consumption of the food product. For instance can the matrix be disrupted by chewing, by the action of saliva or of other gastrointestinal fluids, by peristaltic movements, by the kneading action of the stomach and so on. Suitable carriers encompass liposomes and specific adsorbants, such as cyclodextrins or molecularly imprinted polymers.

Instead of using a solid matrix, the taste essential ingredient and the microorganism and/or enzyme may also be present in different phases of an emulsion or even a double emulsion.

Also combinations of both types, such as encapsulated double emulsions made according to the teaching of *e.g.* EP-1 324 667, may be used, which system guarantees the survival of probiotic bacteria.

Dependent on the type of enzyme and/or probiotic, the optimal activity is achieved at a temperature in the range of 25-60°C. Preferably the enzymes and/or bacteria are selected so that the optimal activity is achieved at a temperature between 35 and 40°C, that is close to body temperature. By encapsulating the enzymes and/or microorganisms, the latter are not in contact with the substrate being present in the food composition, which makes it possible, of course also dependent on the other constituents of the food composition, to store the product at ambient temperature.

Another mechanism for inhibiting the reaction between the microorganism and/or enzyme and the taste essential ingredient is the presence of an inhibitor, and especially a high molecular weight inhibitor, for said reaction. After consumption of the food product encompassing both reactants, the constituents of the food composition are diluted, allowing the interaction between the reactants.

It is also possible to use microorganisms and/or enzymes requiring a specific pH or a specific compound associated with or present in one of the environments in the gastro-intestinal tract, such as a low pH as it exists in the stomach.

In yet a further embodiment the temperature of the food composition is below the temperature needed for the reaction to occur.

The food composition *per se* may also have a pH that is too high or too low for allowing the reaction, which condition changes upon consuming said food composition, for example by dilution. Alternatively, the water activity and/or the salt strength of the surrounding food composition may avoid or inhibit the reaction to occur.

Of course also combinations of the possibilities discussed in the previous paragraphs may be made, such as using a matrix based on calcium phosphate, which salt may lead to an increased pH in the stomach, which increased pH may be associated with an ideal environment for specific microorganisms and/or enzymes.

In another embodiment, the neutralization of the adverse ingredients takes place in the upper intestinal tract, preferably in the oral cavity, pharynx, esophagus and/or stomach.

The most preferred embodiments lead to the conversion of said ingredients having an adverse effect into components that have a beneficial effect in the human or animal body, such as health improving effects.

Generally, it has advantages to use microorganisms instead of enzymes in the food compositions of the present invention. Microorganisms are less expensive than enzymes, and may provide a suitable environment for the reaction of the enzymes prepared by them.

In case probiotics are present in the food compositions of the invention, it is of course of importance that at least part of these probiotics are activated in the guts. That is, it is required that the said bacteria survive the conditions in the stomach. If the probiotics do not have the property of being resistant to the stomach pH, than these may be coated. Alternatively, one can use bacteria that are cultured in an acidic medium to achieve an improved resistance to an acidic pH. By such culture step, the activity of enzymes produced by such bacteria at lower pH values may increase, as well.

Where the first embodiment of the present invention has the taste essential ingredient and the microorganism and/or enzyme present in a food composition, the present invention also relates to a food composition comprising the microorganism and/or enzyme to be used before or after consumption of a food product containing a (potentially present) taste essential ingredient. That is, the food composition comprising the microorganism and/or the enzyme is used profylactically or therapeutically, with the aim of countering the adverse effects of said taste essential ingredients.

If a consumer consumed a particular ingredient which may have adverse effects, or expects to consume such an ingredient, he may consume a food composition comprising a microorganism and/or enzyme to counter the negative effects of such ingredients.

An example of this embodiment of the invention is a food composition, for instance a fermented milk product, such as a yoghurt, buttermilk or cheese (product), comprising transglucosylation enzymes derived from *Lactobacillus,* which enzymes may become active in the gastric environment.

Especially for the embodiment wherein the food composition comprises the microorganism and/or enzyme to be used before consumption of a food product containing a (potentially present) taste essential ingredient, it may be advantageous to delay the passage through the gastro-intestinal tract, and especially through the upper intestinal tract. Such a delay may be achieved by having the microorganisms and/or enzymes present in a controlled release embodiment, such as a film, or in an adhering matrix, such as gel particles that stick to the wall of the oral cavity, or pharynx or esophagus.

Suitable foods, including beverages, incorporating the present invention may be fruit juices, soft drinks, dairy product, candy and chocolate bars, cakes etc. Such products are preferably packed to provide a food composition having an acceptable storage stability and keepability. The steps discussed above to avoid reaction between the enzymes and/or microorganisms and their substrates need to be taken in view of such packed products.

In yet a further aspect, the present invention relates to a process to prepare the food composition of the invention. Such a product may be prepared by adding at any time during the preparation of the food composition, yet preferably after a pasteurization or sterilization step, a supplement containing the enzymes and/or microorganisms capable of converting the taste essential ingredient.

## Claims

1. Food comprising (i) at least one taste essential ingredient that has an adverse effect when taken up in the system of a consumer and (ii) at least one enzyme capable of converting ingredient (i) in a component or components not having said adverse effect or at least having said adverse effect in a decreased degree; wherein ingredient (i) and enzyme (ii) do not react with each other before consumption.

2. Food composition according to claim 1, wherein said taste essential ingredient is an aroma compound, a taste compound or has an effect on the mouthfeel.

3. Food composition according to claim 1 or 2, wherein said taste essential ingredient is selected from the group consisting of a carbohydrate and especially a sugar, a fat, a protein, glutamate, and any mixture thereof.

4. Food composition according to any one of the preceding claims, wherein said taste essential ingredient is a sugar, and said enzyme is selected from the group consisting of the glucosyl transferases, including dextran sucrase; fructosyl transferases, including inulosucrase, levansucrase, fructan:fructan 6G-fructosyltransferase; galactosyl transferase, and mannitoldehydrogenase.

5. Food composition according to any one of the preceding claims, wherein the enzyme is produced by a microorganism present in said food composition.

6. Food composition according to claim 5, wherein the microorganism is a living microorganism, and preferably a probiotic microorganism.

7. Food composition according to claim 5 or 6, wherein the microorganism is selected from the genera *Lactobacillus, Bifidobacterium,* and *Leuconostoc.*

8. Food composition according to claim 5, 6 or 7, wherein the enzyme is an exocellular enzyme.

9. Food composition according to any one of claims 1-4, wherein the enzyme is obtained from or in the form of a lysate of a microorganism.

10. Food composition according to any one of the preceding claims, wherein ingredient (i) and enzyme (ii) do not react with each other before consumption, because the enzyme is encapsulated in a matrix material to be disrupted during or after consumption.

11. Food composition according to any one of the preceding claims, wherein ingredient (i) and enzyme (ii) do not react with each other before consumption, because of the physical and/or chemical conditions in the unconsumed composition are such that the enzyme is not or hardly capable of reacting with ingredient (i).

12. Food composition according to claim 11, wherein the physical and/or chemical conditions are selected from the group consisting of a non-optimal temperature, a reversible enzyme inhibitor, a non-optimal pH, the absence of an activator for the reaction between (i) and (ii), said activator being present in the system of the consumer.

13. Food composition according to any one of the preceding claims, being a dairy product, including a fermented milk product, such as a yoghurt, buttermilk, cheese.
